# EUROPÄISCHE PATENTSCHRIFT

(11) **EP 1 776 064 B1**
(45) Veröffentlichungstag und Bekanntmachung des Hinweises auf die Patenterteilung: **25.08.2010**
(21) Anmeldenummer: 05747275.5
(22) Anmeldetag: 08.06.2005
(51) Int. Cl.: A61F 2/00

(54) **IMPLANTAT ZUR BEHANDLUNG DER MÄNNLICHEN BELASTUNGSHARNINKONTINENZ**
IMPLANT FOR TREATMENT OF MALE URINARY STRESS INCONTINENCE
IMPLANT SERVANT A TRAITER L'INCONTINENCE URINAIRE D'EFFORT MASCULINE

(30) Priorität: 04.08.2004 AT 13412004
(43) Veröffentlichungstag der Anmeldung: 25.04.2007
(73) Patentinhaber: AMI Agency for Medical Innovations GmbH, 6800 Feldkirch (AT)
(72) Erfinder: BAUER, Wilhelm, 1190 Wien (AT)
(74) Vertreter: Sonn & Partner Patentanwälte
(86) Internationale Anmeldenummer: PCT/AT2005/000206
(87) Internationale Veröffentlichungsnummer: WO 2006/012653

(56) Entgegenhaltungen:
- WO-A-00/18319
- WO-A-03/094784
- US-A- 5 934 283

## Beschreibung

Die Erfindung betrifft ein Implantat zur Behandlung der männlichen Belastungsharninkontinenz, mit einem Band, und einem mit einem Fluid befüllbaren Kissen, wobei das Band einen verbreiterten Bereich aufweist, und an diesem Bereich das Kissen angeordnet ist, welches Band mit dem Kissen an die Harnröhre des Patienten gelegt, um den unteren Schambeinast schlingbar und chirurgisch unter Spannung fixierbar ist.

Die Harninkontinenz ist eine häufige Erkrankung, vor allem älterer Personen, beiderlei Geschlechts. Beim Mann stehen als Ursache einer Belastungsharninkontinenz bzw. Stressinkontinenz chirurgische Eingriffe im kleinen Becken, speziell ist hier die radikale Prostatektomie zu nennen, aber auch endourologische Eingriffe an der Prostata und der Harnröhre bei der es zu Verletzungen des Schließmuskels kommen kann, im Vordergrund.

Die sogenannte Belastungsinkontinenz oder Stressinkontinenz ist eine Form der Harninkontinenz, welche durch unwillkürlichen Abgang von Urin bei körperlicher Belastung gekennzeichnet ist. In einem frühen Stadium der Erkrankung kann durch konservative Maßnahmen, wie z.B. Gymnastik zur Stärkung des Beckenbodens oder Biofeedback und Elektrostimulation eine Verbesserung erzielt werden. Bei höhergradiger Inkontinenz ist eine operative Behandlung erforderlich.

Die häufigste chirurgische Behandlung der Belastungsharninkontinenz bei Frauen umfasst eine Implantation eines sogenannten TVT (Tension-free vaginal tape)-Bandes, welches um den hinteren Teil der Harnröhre geschlungen wird und dessen freien Enden oberhalb des Schambeins an der Innenseite der Bauchwand fixiert werden. Dabei bleibt das Kunststoffband spannungsfrei unter der Harnröhre liegen und hindert die Harnröhre bei Belastungen, wie Husten und Niesen, am Tiefertreten und dichtet diese somit ab. Die WO 02/02031 A1 zeigt beispielsweise ein derartiges Implantat zur Behandlung der weiblichen Belastungsharninkontinenz.

Auch die DE 101 03 179 A1 beschreibt eine Vorrichtung zum Behandeln der Belastungsharninkontinenz bei Frauen, umfassend einen Streifen zum Unterstützen der Harnröhre.

Die DE 101 38 950 A1 beschreibt ein TVT-Band zur Behandlung der Harninkontinenz, insbesondere bei der Frau, das flexibel ausgebildet ist und vorzugsweise zumindest teilweise aus resorbierbarem Material besteht. Dieses Band weist eine mit einem Fluid befüllbare Kammer auf. Das Band wird spannungsfrei unter der Harnröhre angeordnet und die freien Enden des Bandes durch einwachsendes Bindegewebe in der Bauchdecke verankert. Eine Eignung zur Behandlung der männlichen Harninkontinenz ist allein aus biomechanischen und anatomischen Gründen nicht möglich.

Neben den oben erwähnten TVT-Bändern werden auch sogenannte TOT(Transobturator tape sling)-Bänder oder transobturatorische Bänder für die chirurgische Therapie für die Belastungsharninkontinenz bei Frauen eingesetzt.

Die US 2003/0212305 A1 beschreibt ein Implantationswerkzeug und eine Implantationsmethode für die Inkontinenzbehandlung bei Frauen mit einem solchen oben genannten transobturatorischen System.

Für die Behandlung der weiblichen Harninkontinenz sind auch Implantate zur Unterstützung der Blase, wie z.B. nach dem US 5, 840, 011 A bekannt.

Der nächstliegende Stand der Technik ist die WO 03/094784 A2. Diese zeigt ein Implantat zur Behandlung der männlichen Belastungsharninkontinenz der gegenständlichen Art, welches einen relativ hohen Aufwand bei der Implantation erfordert.

Für die Anwendung beim Mann sind derartige spannungsfreie Implantate aufgrund der unterschiedlichen anatomischen Gegebenheiten nicht geeignet.

Zur Therapie der männlichen Belastungsharninkontinenz stehen derzeit mit Knochenankern versehene Bänder zur Verfügung, um einen ausreichend hohen Druck auf die Harnröhre ausüben zu können. Dabei wird ein die Harnröhre unterstützendes Band am Beckenknochen fixiert und dadurch ein wirkungsvoller Druck auf die Harnröhre in Belastungssituationen erzielt. Die Fixierung des Bandes am Beckenknochen erfolgt üblicherweise mit Hilfe von Miniaturschrauben bzw. Knochenankern, welche meist aus Titan gefertigt sind. Abgesehen vom aufwendigeren chirurgischen Eingriff stellen die im Knochen verankerten Befestigungselemente Fremdkörper dar, welche zu Abstoßungsreaktionen und Problemen führen können.

Die US 5,163,897 A beschreibt ein Implantat zur Behandlung der männlichen Belastungsharninkontinenz, wobei ein aufblasbarer Ballon entlang der Harnröhre platziert und durch entsprechende klauenartige Elemente am Schwellkörper befestigt wird. Der Ballon ist über eine Leitung mit einer Handpumpe verbunden, welche im Hodensack angeordnet ist. Durch Betätigen der Handpumpe kann der Ballon aufgeblasen werden, so dass ein entsprechender Druck auf die Harnröhre ausgeübt wird, welcher diese verschließt. Zum Zwecke der Blasenentleerung muss der Ballon durch Betätigung eines entsprechenden Ventils entleert werden, so dass kein Druck auf die Harnröhre mehr ausgeübt wird und somit einen Durchfluss von Harn ermöglicht wird. Abgesehen von der relativ hohen Komplexität des Implantats und dem hohen Aufwand der Implantation erfordert dieses System die aktive Mitarbeit des Patienten, was insbesondere im Falle älterer Patienten auf Probleme stößt.

Die Aufgabe der vorliegenden Erfindung besteht in der Schaffung eines oben erwähnten Implantats, durch welches die männliche Belastungsharninkontinenz wirkungsvoll behandelt werden kann und welches möglichst einfach und ohne Nebenwirkungen implantiert werden kann. Das Implantat soll möglichst einfach und kostengünstig aufgebaut sein und möglichst wenig Abstoßungsreaktionen hervorrufen. Nachteile bekannter Implantate sollen vermieden bzw. reduziert werden.

Die Aufgabe der vorliegenden Erfindung wird dadurch gelöst, dass die freien Enden des Bandes im implantierten Zustand nach der Umschlingung am verbreiterten Bereich fixierbar sind, und das Kissen mit einer außerhalb des Bandes verlaufenden Leitung zur Zu- und Abführung des Fluids verbunden ist. Das Band mit dem verbreiterten Bereich und dem Kissen wird chirurgisch so eingesetzt, dass das Kissen über den verbreiterten Bereich des Bandes einen entsprechenden Druck auf die Harnröhre ausübt, der gerade so stark ist, dass es aufgrund von Belastungen, wie z.B. Husten und Niesen zu keinem Harnaustritt kommt. Andererseits kann der Druck über das Kissen individuell so eingestellt werden, dass ohne Betätigung am Implantat oder Vornahme anderer Maßnahmen durch den Patienten eine Entleerung der Blase durchgeführt werden kann. Durch den verbreiterten Bereich des Bandes wird über einen längeren Abschnitt der Harnröhre ein Druck auf diese ausgeübt. Durch die Druckverteilung über dem verbreiterten Bereich kann es zu keinen Schädigungen der Harnröhre kommen. Das erfindungsgemäße Implantat benötigt keine Fixierung am Beckenknochen, sondern wird durch das Anlegen an die Harnröhre und Durchführen der freien Bandenden durch die Öffnungen im Beckenknochen in der gewünschten Lage platziert und durch Fixierung der Bandenden am verbreiterten Bereich eine dauerhafte Fixierung am Becken erzielt. Durch den Wegfall der Befestigungselemente am Beckenknochen werden Abstoßungsreaktionen und Operationskomplikationen vermieden. Darüber hinaus ist die Implantation rascher und einfacher und somit mit weniger Belastung für den Patienten durchführbar. Durch die Anordnung des Kissens ist das vorliegende Implantat optimal auf die jeweiligen Gegebenheiten einstellbar, indem das Kissen zur Ausübung eines höheren Drucks auf die Harnröhre mit dem Fluid befüllt wird oder zur Reduktion des auf die Harnröhre ausgeübten Drucks eine entsprechende Menge an Fluid aus dem Kissen abgezogen wird. Diese Einstellung kann auch besonders einfach und rasch nach der Implantation durch Befüllung oder Absaugung des Kissens über die Haut erfolgen. Um die nachträgliche Befüllung des Kissens mit dem Fluid oder eine Absaugung des Fluids aus dem Kissen leichter zu ermöglichen, kann das Kissen mit einer Leitung verbunden, welche außerhalb des Bandes verläuft. Die Leitung wird beispielsweise im Hodensack des Patienten platziert und kann vom behandelten Arzt leicht ertastet werden und schließlich das Fluid durch Durchstechen der Haut und Durchstechen der Leitung in das Kissen eingebracht oder aus dem Kissen abgesaugt werden. So kann jederzeit eine nachträgliche Korrektur des Druckes auf die Harnröhre für optimale Behandlungsergebnisse vorgenommen werden. Das beschriebene Implantat zur Behandlung der männlichen Belastungsharninkontinenz benötigt keine Schrauben mehr und ist selbsttragend ausgebildet.

Der verbreiterte Bereich des Bandes ist vorteilhafterweise im Wesentlichen in der Längsmitte des Bandes angeordnet. Die Länge des Bandes ist so gewählt, dass dieses je nach anatomischen Verhältnissen jeweils einmal um den Beckenknochen geschlungen werden kann und danach die freien Enden des Bandes am verbreiterten Bereich, welcher an der Harnröhre anliegt beispielsweise durch Anlegen einer Naht oder durch Verklebung fixiert werden können. Die Enden des Bandes können auch während der Implantation auf die gewünschte Länge abgeschnitten werden.

Das mit dem Fluid befüllbare Kissen ist vorteilhafterweise an jener Seite des Bandes angeordnet, welche im implantierten Zustand der Harnröhre zugewandt ist.

Vorzugsweise weist der verbreiterte Bereich zumindest eine Öffnung für das freie Ende des Bandes auf, wodurch die Befestigung des Bandes erleichtert werden kann.

Vorteilhafterweise ist das freie Ende der Leitung verschlossen. Die Leitung bzw. das freie Ende der Leitung ist für ein möglichst leichtes Durchstechen mit einer entsprechenden Nadel vorteilhafterweise mit einer dünnen Membran versehen.

Um ein Einstechen in das freie Ende der Leitung zu erleichtern, kann dieses verbreitert ausgebildet sein.

Vorteilhafterweise wird als Fluid physiologische Kochsalzlösung verwendet, welche auch im Falle eines Undichtwerdens des Kissens keinerlei negative Auswirkungen auf den Körper hat.

Das Kissen kann mit dem Band an der entsprechenden Seite des verbreiterten Bereichs mit diesem verklebt sein. Dabei werden entsprechende Kleber verwendet, welche keine Abstoßungsreaktionen hervorrufen und auch bei Implantation über längere Zeit ihre Klebewirkung nicht verlieren.

Ebenso ist es möglich, dass das Kissen und das Band einstückig hergestellt sind.

Vorteilhafterweise ist das Band aus einem Kunststoffgewebe, insbesondere einem Polypropylengewebe hergestellt. Diese Materialien haben bereits bei anderen Implantaten hervorragende Eigenschaften gezeigt.

Das Gewebeband kann in Silikon getränkt sein.

Das Kissen ist gemäß einem weiteren Merkmal der Erfindung aus einem elastischen Kunststoff, insbesondere Silikon gebildet.

Um eine leichtere Fixierung der Enden des Bandes nach der Implantation am verbreiterten Bereich zu ermöglichen, können die Enden des Bandes verjüngend ausgebildet sein.

Zur weiteren Erleichterung der Implantation können an den Enden des Bandes Ösen oder dgl. zur Nahtfixation vorgesehen sein.

Die Erfindung betrifft ebenso eine Methode zur Implantation des beschriebenen Implantats, welche anhand der beigefügten Zeichnungen, welche ein Ausführungsbeispiel des Implantats zur Behandlung der männlichen Belastungsharninkontinenz zeigt, näher erläutert wird.

Darin zeigen:
- Fig. 1: eine Ansicht auf ein Band im ausgebreiteten flachen Zu- stand;
- Fig. 2: eine Detailansicht auf das Band gemäß Fig. 1 mit daran angeordnetem Kissen;
- Fig. 3: ein Schnittbild durch das Band gemäß Fig. 2 entlang der Schnittlinie III-III;
- Fig. 4: schematisch die Ansicht auf das am Becken befestigte Im- plantat nach der Implantation in der Ansicht von unten; und

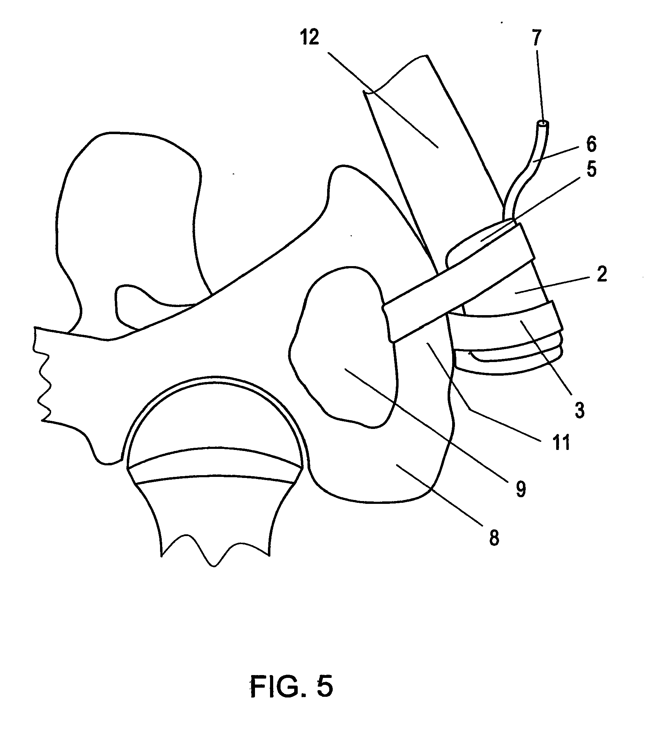

- Fig. 5: das am Becken befestigte Implantat in Seitenansicht.

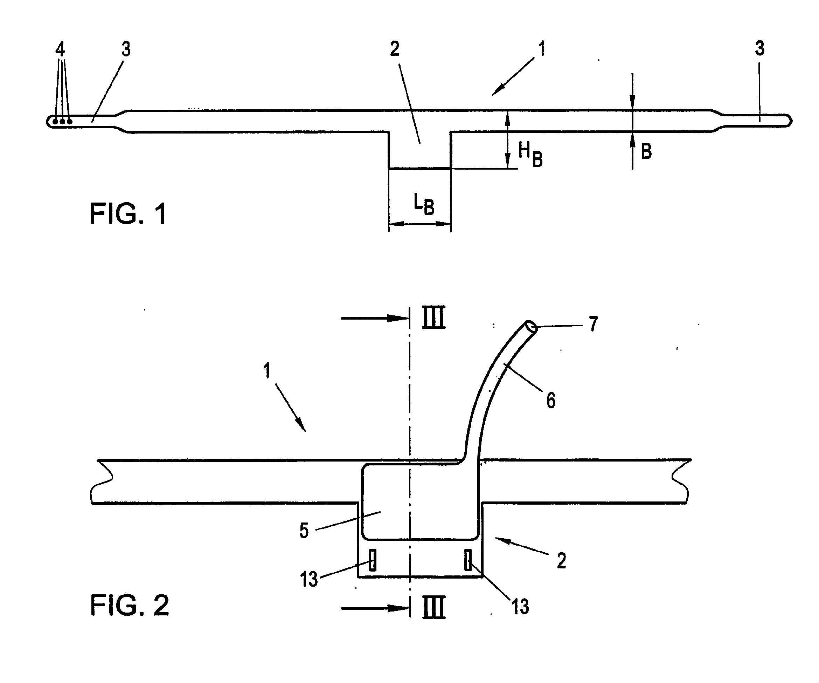

Fig. 1 zeigt das Band 1 zur Bildung des Implantats zur Behandlung der männlichen Belastungsharninkontinenz, welches im Wesentlichen in der Längsmitte einen verbreiterten Bereich 2 aufweist. Die freien Enden 3 des Bandes 1 können verjüngend ausgebildet sein. Zur Nahtfixation können an den freien Enden 3 Ösen 4 oder dgl. angeordnet sein. Das Band 1 besteht vorzugsweise aus einem Kunststoffgewebe, insbesondere Polypropylengewebe, welches beispielsweise in Silikon getränkt sein kann. Diese Materialien weisen gute Biokompatibilität auf und werden in der Chirurgie häufig verwendet. Weiters sind derartige Materialien relativ kostengünstig herstellbar. Die Dimensionen des erfindungsgemäßen Bandes 1 werden an die jeweiligen Gegebenheiten angepasst. Die Gesamtlänge L des Bandes 1 kann beispielsweise im Bereich zwischen 500 und 600 mm liegen. Der verbreiterte Bereich 2 des Bandes 1 weist typischerweise eine Länge L_{B} gleich 50 mm und eine Höhe H_{B} von 40 mm auf. Die Breite B des Bandes 1 ist typischerweise 15 mm. Das Band 1 wird im minimalchirurgischen Verfahren implantiert, in dem die Harnröhre durch einen kleinen Schnitt freigelegt wird und das Band 1 mit entsprechenden Implantationswerkzeugen, welche beispielsweise für die Implantation der transobturatorischen Bänder zur Behandlung der weiblichen Harninkontinenz bekannt sind, eingebaut wird.

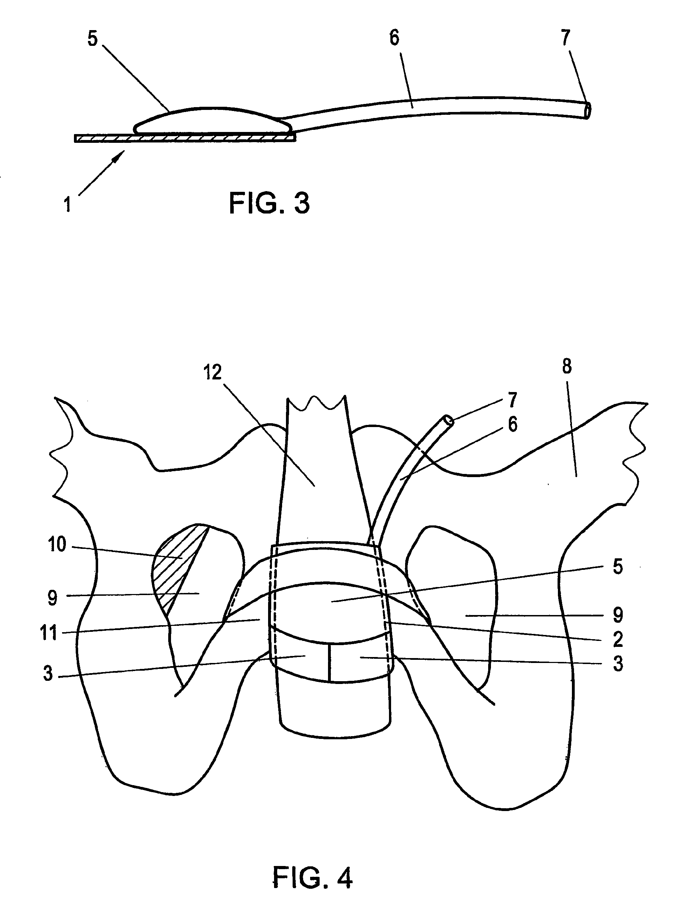

Fig. 2 und 3 zeigen ein Detail des Bandes 1 im Bereich des verbreiterten Bereichs 2, wobei ein Kissen 5 zumindest über einen Teil des verbreiterten Bereichs 2 vorgesehen ist. Am verbreiterten Bereich 2, über welchem kein Kissen 5 platziert ist, können Öffnungen 13 angeordnet sein, in welche die freien Enden 3 des Bandes 1 zur besseren Fixierung eingesteckt werden können. Ebenso ist es möglich, dass mehrere miteinander verbundene Kissen 5, angeordnet sind. Das Kissen 5 kann mit dem Band 1 verklebt oder einstückig mit diesem hergestellt sein. Zur leichteren nachträglichen Befüllung oder Entleerung des Kissens 5 ist dieses mit einer Leitung 6 verbunden, dessen freies Ende 7 verschlossen ist, so dass das im Kissen 5 angeordnete Fluid, insbesondere die Kochsalzlösung, nicht ungewollt austreten kann. Das freie Ende 7 der Leitung 6 wird an einer geeigneten Stelle beispielsweise im Bereich des Hodensackes des Patienten angeordnet. Durch einen Stich durch die Haut und das freie Ende 7 der Leitung 6 kann das Fluid mit Hilfe einer Spritze in das Kissen 5 eingebracht werden bzw. aus diesem abgesaugt werden.

Fig. 4 zeigt eine Ansicht auf das am Becken 8 befestigte Implantat nach der Implantation. Die Implantation kann in Spinalanästhesie oder Allgemeinnarkose durchgeführt werden. Der Patient wird in Rückenlage perineal gelagert. Es erfolgt ein vertikaler perinealer Schnitt knapp unterhalb des Skrotums in einer Länge von ca. 5 cm. Daraufhin wird die Harnröhre und der darüberliegende Musculus bulbospongiosus dargestellt. An dieser Struktur 12 wird beiderseits lateral der untere Schambeinast 11 (Ramus inferior ossis pubis) dargestellt. Es finden sich als anatomische Strukturen von lateral ausgehend der Ramus inferior ossis pubis 11, das Crus des Corpus cavernosus und medial anliegend die Harnröhre mit dem Musculus bulbospongiosus, der erhalten wird (Struktur 12). Danach erfolgt ca. 2 cm unterhalb der Symphyse eine kleine Inzision der pelvinen Faszie lateral des Crus und medial des Ramus inferior ossis pubis 11 mit der Schere an beiden Seiten. Durch diese Inzision wird ein helikaler Trokar, ähnlich dem wie in Patent WO 02/02031 A1 beschrieben um den Ramus inferior ossis pubis 11 geführt. Dadurch wird die Membrana obturatoria nahe dem Ramus inferior ossis pubis 11, auf der kontralateralen Seite vom Verlauf des Nervus obturatorius im Bereich 10 in der Öffnung 9 (dem Foramen obturatorium) des Beckens 8 durchstoßen. Dabei wird der gefährliche Bereich 10 der Öffnungen 9 im Becken 8, in welchem Nerven und Blutgefäße verlaufen nicht in Mitleidenschaft gezogen, wodurch das Operationsrisiko und die Blutungsgefahr reduziert werden kann. Im Gegensatz zur herkömmlichen Implantationstechnik bei der Frau wird der Trokar in einer sogenannten "inside-out Technik" geführt und die Haut nicht durchstoßen, sondern der Trokar wird von dorsal kommend an der ventralen Seite des Ramus inferior ossis pubis 11 zur perinealen Inzisionsstelle geführt. Durch die Öse im Trokar wird ein Ende des Bandes 1 fixiert und der Trokar wird in einer helikalen Bewegung, die durch die Krümmung vorgegeben ist, zurückgezogen. Derselbe Implantationsweg erfolgt ebenso an der anderen Seite. Durch diesen Implantationsweg kommt das mittig des Bandes 1 im verbreiterten Bereich 2 angeordnete Kissen 5 vor der Harnröhre in der Struktur 12 in der Höhe der Symphyse zu liegen. Die Enden der Schlinge werden nun am unteren Teil des verbreiterten Bereichs 2 des Bandes 1 mittels Naht oder durch Kleben fixiert. Durch Befüllen des Kissens 5 und gleichzeitigem retrograden Messens des urethralen Verschlussdrucks wird der optimale Druck auf die Harnröhre in der Struktur 12 eingestellt. Danach wird das freie Ende 7 der Leitung 6 zur Befüllung des Kissens 5 in eine kleine chirurgisch geschaffene Tasche in den Hodensack gelegt. Danach erfolgt der schichtweise Wundverschluss.

Die Befestigung des Implantats erfolgt somit ohne Verankerungen am unteren Schambeinast 11 des Beckens 8, was die Operation erleichtert und die Risken minimiert. Während der Implantation wird der Druck auf die Harnröhre in der Struktur 12 durch entsprechende Befestigung der freien Enden 3 des Bandes 1 und durch Zu- oder Abfuhr des Fluids in bzw. aus dem Kissen 5 erzielt. Durch Befüllung des Kissens 5 über die Leitung 6 bzw. das freie Ende 7 der Leitung 6 wird ein entsprechender Druck auf die Harnröhre ausgeübt, der eine Abdichtung bei Belastungen, wie z.B. Husten und Niesen sichert und dennoch eine bewusste Blasenentleerung zulässt. Dieser gewünschte Druck wird, wie oben beschrieben, während der Operation gemessen und durch entsprechendes Auffüllen des Kissens 5 eingestellt. Nachträglich kann eine Anpassung beispielsweise an geänderte Situationen durch Befüllen oder Entleeren des Kissens 5 über die Leitung 6 jederzeit und ambulant ohne viel Aufwand erfolgen.

Die vorliegende Erfindung zeigt ein neues transobturatorisches System zur Behandlung der männlichen Belastungsharninkontinenz, welches keine Schrauben zur Verankerung am Becken benötigt. Das Implantat ist selbsttragend ausgeführt und ist durch die Integration des Kissens individuell einstellbar.

## Patentansprüche

1. Implantat zur Behandlung der männlichen Belastungsharninkontinenz, mit einem Band (1), und einem mit einem Fluid befüllbaren Kissen (5), wobei das Band (1) einen verbreiterten Bereich (2) aufweist, und an diesem Bereich (2) das Kissen (5) angeordnet ist, welches Band (1) mit dem Kissen (5) an die Harnröhre des Patienten gelegt, um den unteren Schambeinast schlingbar und chirurgisch unter Spannung fixierbar ist, **dadurch gekennzeichnet, dass** die freien Enden (3) des Bandes (1) im implantierten Zustand nach der Umschlingung am verbreiterten Bereich (2) fixierbar sind, und das Kissen (5) mit einer außerhalb des Bandes (1) verlaufenden Leitung (6) zur Zu- und Abführung des Fluids verbunden ist.

2. Implantat nach Anspruch 1, **dadurch gekennzeichnet, dass** der verbreiterte Bereich (2) im Wesentlichen in der Längsmitte des Bandes (1) angeordnet ist.

3. Implantat nach Anspruch 1 oder 2, **dadurch gekennzeichnet, dass** das Kissen (5) an jener Seite des Bandes (1) angeordnet ist, welche im implantierten Zustand der Harnröhre zugewandt ist.

4. Implantat nach einem der Ansprüche 1 bis 3, **dadurch gekennzeichnet, dass** der verbreiterte Bereich (2) zumindest eine Öffnung (13) zur Aufnahme der freien Enden (3) des Bandes (1) aufweist.

5. Implantat nach einem der Ansprüche 1 bis 4, **dadurch gekennzeichnet, dass** das freie Ende (7) der Leitung (6) verschlossen ist.

6. Implantat nach Anspruch 5, **dadurch gekennzeichnet, dass** das freie Ende (7) der Leitung (6) verbreitert ausgebildet ist.

7. Implantat nach einem der Ansprüche 1 bis 6, **dadurch gekennzeichnet, dass** das Fluid durch Kochsalzlösung gebildet ist.

8. Implantat nach einem der Ansprüche 1 bis 7, **dadurch gekennzeichnet, dass** das Kissen (5) mit dem Band (1) verklebt ist.

9. Implantat nach einem der Ansprüche 1 bis 7, **dadurch gekennzeichnet, dass** das Kissen (5) und das Band (1) einstückig hergestellt sind.

10. Implantat nach einem der Ansprüche 1 bis 9, **dadurch gekennzeichnet, dass** das Band (1) aus einem Kunststoffgewebe, insbesondere Polypropylengewebe besteht.

11. Implantat nach Anspruch 10, **dadurch gekennzeichnet, dass** das Band (1) in Silikon getränkt ist.

12. Implantat nach einem der Ansprüche 1 bis 11, **dadurch gekennzeichnet, dass** das Kissen (5) aus einem elastischen Kunststoff, insbesondere Silikon gebildet ist.

13. Implantat nach einem der Ansprüche 1 bis 12, **dadurch gekennzeichnet, dass** die Enden (3) des Bandes (1) verjüngend ausgebildet sind.

14. Implantat nach einem der Ansprüche 1 bis 13, **dadurch gekennzeichnet, dass** an den Enden (3) des Bandes (1) Ösen (4) zur Nahtfixation vorgesehen sind.

## Claims

1. An implant for treating male urinary stress incontinence, comprising a tape (1) and a cushion (5) capable of being filled with fluid, wherein the tape (1) has a broadened region (2), to which region (2) the cushion (5) is arranged, which tape (1) with the cushion (5), when laid onto the patient's urethra, may be looped over the lower pubic branch and may be surgically fixed under tension, **characterised in that** the free ends (3) of the tape (1), when being implanted, after the loop-over, may be fixed to said broadened region (2), and **in that** the cushion (5) is connected with a duct (6), which extends outside the tape (1), for supplying and removing the fluid.

2. The implant according to claim 1, **characterised in that** the broadened region (2) is arranged substantially in the longitudinal center of the tape (1).

3. The implant according to claim 1 or 2, **characterised in that** the cushion (5) is arranged on that side of the tape (1) facing the urethra in the implanted state.

4. The implant according to any one of claims 1 to 3, **characterised in that** the broadened region (2) comprises at least one opening (13) for receiving the free ends (3) of the tape (1).

5. The implant according to any one of claims 1 to 4, **characterised in that** the free end (7) of the duct (6) is closed.

6. The implant according to claim 5, **characterised in that** the free end (7) of the duct (6) is designed to be broadened.

7. The implant according to any one of claims 1 to 6, **characterised in that** the fluid is formed by saline solution.

8. The implant according to any one of claims 1 to 7, **characterised in that** the cushion (5) is glued with the tape (1).

9. The implant according to any one of claims 1 to 7, **characterised in that** the cushion (5) and the tape (1) are produced integrally.

10. The implant according to any one of claims 1 to 9, **characterised in that** the tape (1) consists of a synthetic fabric, in particular of polypropylene fabric.

11. The implant according to claim 10, **characterised in that** the tape (1) is soaked in silicone.

12. The implant according to any one of claims 1 to 11, **characterised in that** the cushion (5) is formed by an elastic synthetic material, in particular silicone.

13. The implant according to any one of claims 1 to 12, **characterised in that** the ends (3) of the tape (1) are designed to taper.

14. The implant according to any one of claims 1 to 13, **characterised in that** eye hooks (4) are provided on the ends (3) of the tape (1) for fixing the suture.

## Revendications

1. Implant pour le traitement de l'incontinence urinaire d'effort masculine, comportant une bande (1) et une poche (5) pouvant être remplie avec un fluide, la bande (1) comportant une zone élargie (2), et la poche (5) étant disposée dans cette zone (2), ladite bande (1) étant située avec la poche (5) au niveau de l'urètre du patient, pour pouvoir être enroulée autour du rameau pubien et y être fixée sous contrainte par chirurgie, **caractérisé en ce que** les extrémités libres (3) de la bande (1) en position implantée peuvent être fixées sur la zone élargie (2) après enroulement, et la poche (5) est reliée à une conduite (6) qui s'étend à l'extérieur de la bande (1) pour l'admission et l'évacuation du fluide.

2. Implant selon la revendication 1, **caractérisé en ce que** la zone élargie (2) est disposée sensiblement au milieu de la dimension longitudinale de la bande (1).

3. Implant selon la revendication 1 ou 2, **caractérisé en ce que** la poche (5) est disposée sur le côté de la bande (1) qui, dans la position implantée, est orienté vers l'urètre.

4. Implant selon l'une quelconque des revendications 1 à 3, **caractérisé en ce que** la zone élargie (2) comporte au moins une ouverture (13) destinée à recevoir les extrémités libres (3) de la bande (1).

5. Implant selon l'une quelconque des revendications 1 à 4, **caractérisé en ce que** l'extrémité libre (7) de la conduite (6) est fermée.

6. Implant selon la revendication 5, **caractérisé en ce que** l'extrémité libre (7) de la conduite (6) est réalisée sous forme élargie.

7. Implant selon l'une quelconque des revendications 1 à 6, **caractérisé en ce que** le fluide est formé par une solution de chlorure de sodium.

8. Implant selon l'une quelconque des revendications 1 à 7, **caractérisé en ce que** la poche (5) est collée à la bande (1).

9. Implant selon l'une quelconque des revendications 1 à 7, **caractérisé en ce que** la poche (5) et la bande (1) sont réalisées d'une seule pièce.

10. Implant selon l'une quelconque des revendications 1 à 9, **caractérisé en ce que** la bande (1) est réalisée dans un tissu de matière plastique, en particulier un tissu de polypropylène.

11. Implant selon la revendication 10, **caractérisé en ce que** la bande (1) est imprégnée de silicone.

12. Implant selon l'une quelconque des revendications 1 à 11, **caractérisé en ce que** la poche (5) est réalisée dans une matière plastique élastique, en particulier en silicone.

13. Implant selon l'une quelconque des revendications 1 à 12, **caractérisé en ce que** les extrémités (3) de la bande (1) sont amincies.

14. Implant selon l'une quelconque des revendications 1 à 13, **caractérisé en ce que** des oeillets (4) pour la fixation par couture sont prévus au niveau des extrémités (3) de la bande (1).
